# EUROPEAN PATENT APPLICATION

(11) **EP 2 682 750 A1**
(43) Date of publication of application: **08.01.2014**
(21) Application number: 12174539.2
(22) Date of filing: 02.07.2012
(51) Int. Cl.: G01N 33/574, G01N 33/50

(54) **In vitro method for the diagnosis and surveillance of cancer**

(71) Applicant: Sotio a.s., 170 00 Prague 7 (CZ)
(72) Inventor: Bartunková, Jirina, 110 00 Praha 1 (CZ); Spísek, Radek, 148 00 Praha 4 (CZ)
(74) Representative: Keller, Günter

(57) **Abstract**

The present invention relates to an in vitro method for the diagnosis and/or the monitoring and/or predicting the progress of a cancer disease, characterized in that in at least one sample the ratio of Tregs-cells to at least one other group of T cells comprising the group of Th17 cells, Th1 cells and/or Th2 cells is determined.

## Description

### Field of invention

The present invention demonstrates an alteration in the tumor-infiltrating immune cell pattern during cancer, in particular ovarian cancer development and clearly shows a dynamic shift from an active anti-tumor immune response represented by the presence of effector T cells, which prevail over Tregs in the early stages of epithelial ovarian cancer (EOC), to a significant immune suppression in the advanced stages of disease.

The type of immune cells that are present within the tumor microenvironment can play a crucial role in the survival of patients. However, little is known about the dynamics of the tumor-infiltrating immune cells during disease progression. The immune cells that infiltrated the tumor tissues of ovarian cancer patients have been studied at different stages of disease. The early stages of development of ovarian carcinomas were characterized by a strong Th17 immune response, whereas in stage II patients, recruitment of high numbers of Th1 cells was observed.

In disseminated tumors (stage III-IV), a dominant population of Helios⁺ activated regulatory T cells (Tregs) was detected along with high numbers of macrophages and myeloid dendritic cells (mDCs). Tumor-infiltrating Tregs had markedly lower expression of CCR4 than circulating Tregs, and the numbers of tumor-infiltrating Tregs significantly correlated with the levels of CCL22 in ovarian tumor cell culture supernatants, suggesting their recruitment via a CCR4/CCL22 interaction. CCL22 was mainly produced by tumor cells, macrophages and mDCs in the primary ovarian tumors, and its expression markedly increased in response to IFNγ. Taken together, the specific recruitment of Tregs, probably triggered by inflammatory stimuli, leads to a significant immune suppression in the advanced stages of ovarian cancer.

The above-summarized finding is used in an in vitro method for the diagnosis and surveillance of various cancers, in particular of ovarian cancer. The method makes use in identifying different cell types, preferably by using markers on the surface of the cells. T cell mediated immunity comprises a variety of different T cells. Cytotoxic T cells are for example characterized by the surface antigen CD8 (MHC class I receptor). The T helper cells can be divided into several subgroups. The for the present invention most relevant T cells have the surface marker CD4⁺ (MHC class II) and comprise in particular CD4⁺ Th17 cells which enhance the neutrophil response and are known to be effective against in particular extracellular bacteria. It is assumed that Th17 cells have in tumors the effect to kill tumor cells. Another interesting group of cells is called Treg which are CD4⁺ regulatory T cells. This type of cells suppresses the T cell responses. Usually this kind of cells has the effect to avoid an exaggerated immune response. In tumor cells, however, it is assumed that the presence of Treg cells regulates down the own immune defense of the body against tumor cells and therefore eventually enhance tumor growth.

### Background of the invention

Ovarian cancer is one of the ten most common malignancies in females worldwide and is known to have the highest mortality rate among gynecologic cancers. Because of the lack of sensitive and specific biomarkers and due to the fact that the disease tends to develop and spread rapidly, almost 70% of patients are diagnosed at an advanced stage of tumor dissemination with poor prognosis. Although conventional treatment leads to a significant reduction in malignant cell numbers in more than 80% of ovarian carcinomas, most of the patients experience an eventually lethal relapse of the disease within 2-5 years because of the persistence of a small number of chemotherapy-resistant tumor cells. To improve the prognosis of ovarian cancer patients and for a better adaptation of tumor therapy, there is a need for superior prognostic markers and novel therapeutic strategies that can identify high-risk patients and diminish the likelihood of relapse, respectively.

Immune surveillance has been proposed to play a crucial role in cancer development and progression. Experiments in immunodeficient mice have shown that the immune system is able to recognize and eradicate tumors. However, although cancer cells can prime tumor-specific immune responses, interactions between the tumor and the host immune system may not result in clinical regression of the disease but instead to the development of an immunosuppressive microenvironment within the tumor tissue, consequently promoting immune evasion. Moreover, it is not sure that results experimentally obtained from laboratory mice are also valid in cancer patients.

Despite being unable to eradicate established tumors, the presence of certain tumor-infiltrating immune cells can represent strong prognostic markers in cancer patients. It has been reported that high numbers of tumor-infiltrating CD3⁺ T cells are associated with a favorable clinical outcome in advanced ovarian cancer patients. More recent studies have reported that improved survival is associated with enhanced numbers of CD8⁺ cytotoxic T cells. By contrast, high numbers of CD4⁺CD25⁺FoxP3⁺ regulatory T cells (Tregs), plasmacytoid dendritic cells, and B7-H4⁺ macrophages within the tumor tissue can indicate a poor prognosis. The role of CD4⁺ T helper cells is not well documented; however, there is strong evidence that Th17 cells may be substantial players in cancer immunity.

Proinflammatory Th17 cells have primarily been associated with autoimmune diseases and mucosal immunity. It has been demonstrated that Th17 cells are present in different types of tumors, including ovarian cancer, head and neck cancer, gastric cancer, breast cancer, colorectal cancer, and prostate cancer. Although extensively studied, the exact role of Th17 cells in tumor immunity and the survival of patients remains controversial. On the one hand, IL-17-producing cells have been reported to promote antitumor immunity, but on the other hand, IL-17 is known to act as an angiogenic factor that can enhance tumor growth.

Most published studies have focused on the pattern and prognostic significance of different types of tumor-infiltrating immune cells; however, little is known about the dynamics of the immune response within the tumor tissue during disease progression. The distribution, phenotype and clinico-pathological significance of CD4⁺ and CD8⁺ T lymphocytes, dendritic cells (DCs) and macrophages in different stages of epithelial ovarian cancer (EOC) has been evaluated. The results of the present invention demonstrate an alteration in the tumor-infiltrating immune cell pattern during cancer development and clearly show a dynamic shift from an effector Th17 cell infiltration, which prevail over Tregs in the early stages of EOC, to a significant Treg accumulation in the advanced stages of disease.

### Preferred embodiments of the present invention

In the present disclosure several terms are used which are commonly used in the field of immunology. For the meaning of the terms we refer therefore in particular to the following textbooks:
- Janeway's Immunobiology, 7th edition, Kenneth Murphy, Paul Travers, Mark Walport (2008)
- Fundamental Immunology, 6th edition, William E. Paul (2008), Wolters Kluwer, or
- Pezzutto, Ulrichs, Burmester; Taschenatlas der Immunologie, Thieme, 2nd edition (2007),
which comprise an extensive list of CD markers and explanations thereof, cytokines and the commonly used abbreviations thereof.

In the course of the present invention the following observations were made:

### A) Th17 lymphocytes accumulate in the tumor tissue and their proportions negatively correlate with disease progression

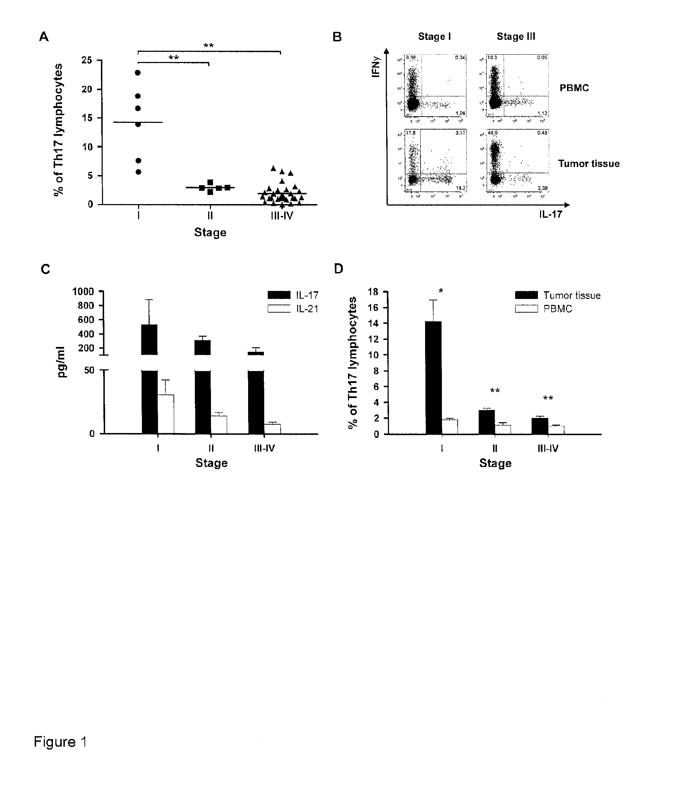

Th17 lymphocytes were detected in 100% of the analyzed ovarian tumor samples (range = 0.06 - 22.8%). The proportion of Th17 cells within the CD4⁺ T cell subset was significantly higher in the tumor tissues obtained from patients with limited disease (FIGO stage I) than in those from patients suffering from disseminated disease (FIGO stage III-IV) (see Fig. 1A, B). The frequency of Th17 cells within the whole tumor-derived single cell suspensions showed the same pattern, indicating that the absolute numbers of Th17 cells significantly decreased in the advanced disease. In accord with these findings, the concentration of IL-17 and IL-21 in tumor tissue-derived cell culture supernatants of early stage patients was markedly higher in both unstimulated and PMA- and ionomycin-stimulated (see Fig. 1 C) cultures. Despite the significant decrease in Th17 cell proportions in the later stages of the disease, the percentage of tumor-infiltrating Th17 cells was still significantly higher when compared with the peripheral blood of patients (see Fig. 1D). The frequency of Th17 cells in the peripheral blood alone was not predictive of the number or proportion of Th17 cells in the tumor tissue. It can be used, however, for control purposes.

### B) The proportion of tumor-infiltrating Tregs is enhanced in the tumor tissue and increases with disease progression

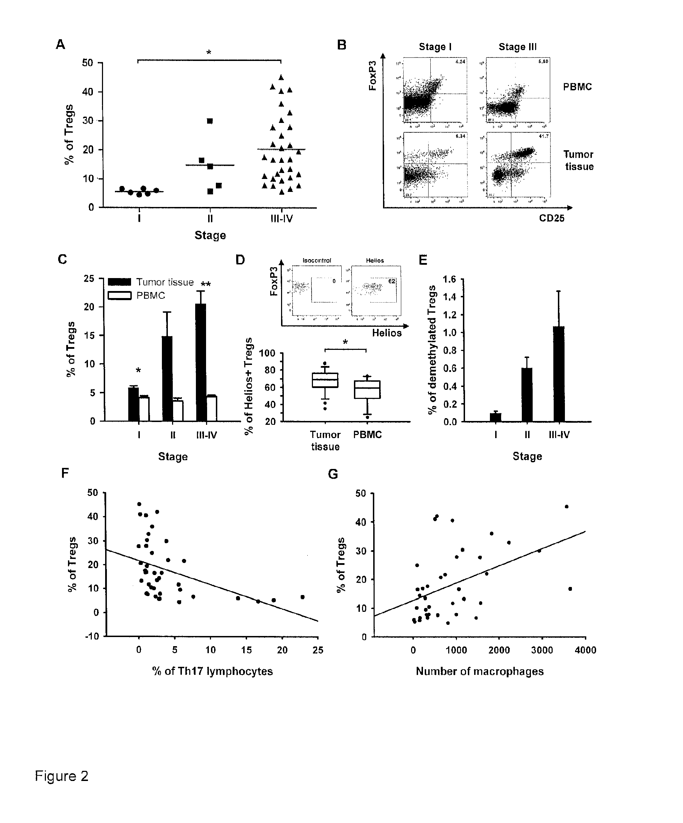

In contrast to Th17 cells, the proportion of CD4⁺CD25⁺CD127-FoxP3⁺ Tregs was significantly higher in the tumor tissues obtained from patients with disseminated disease (FIGO stage III-IV) when compared with the samples from patients with stage I disease (see Fig. 2A, B). The proportion of tumor-infiltrating Tregs (Ti-Tregs) was significantly higher than that found in the peripheral blood of patients in all stages of the disease (see Fig. 2C). To better characterize the status of Ti-Tregs, the transcription factor, Helios, which is a marker of Treg activation was also analyzed. The proportion of Helios⁺ Tregs was significantly higher in the tumor tissue (67.8 ± 2.5%) than in the peripheral blood (56.3 ± 4.2%) of EOC patients (see Fig. 2D). There was no difference in the proportions of Helios⁺ Tregs in the different stages of disease. The increasing proportion of stable Ti-Tregs during disease progression was further confirmed using a quantitative real time PCR-based methylation assay, which assessed the percentage of stable Tregs that were demethylated at the *FoxP3* TSDR in tumor tissue-derived single cell suspensions (see Fig. 2E). The proportions of Tregs and Th17 lymphocytes were inversely correlated in the same tumors (r = -0.44, p < 0.01) (see Fig. 2F), whereas the proportion of Tregs positively correlated with the number of macrophages (r = 0.47, p < 0.01) (Fig. 2G).

### C) Proportions of Th1 and CD8⁺ T lymphocytes, dendritic cells (DCs) and macrophages do not show significant changes during disease progression

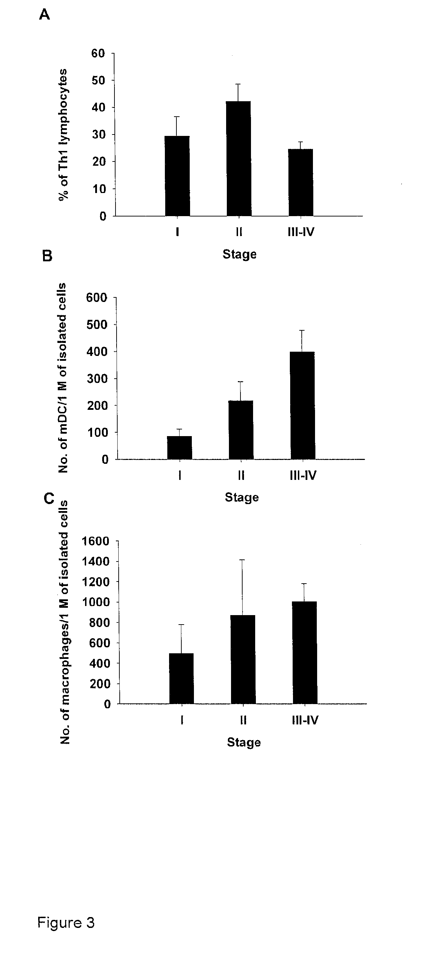

In addition to Th17 cells and Tregs, the presence of other immune cell subsets, including Th1 cells, CD8⁺ T cells, pDCs, myeloid Dendritic Cells (mDCs) and macrophages, was analyzed in the tumor tissue. Although there was a trend toward an increase in mDC and macrophage proportions in the advanced tumors (see Fig. 3), none of these cell types showed any statistically significant dynamics during ovarian cancer progression.

### D) Cytokine and chemokine profiles of tumor-derived single cell suspensions support the recruitment of polarized Th lymphocytes into the tumor tissue rather than their in situ priming and polarization

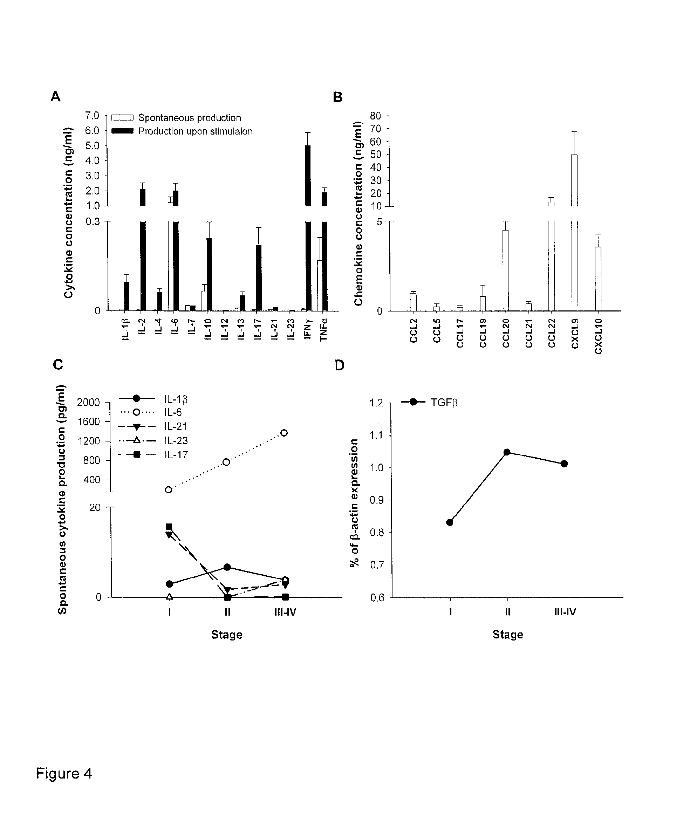

To better characterize the tumor microenvironment and to evaluate, whether the cytokine profile supports *in situ* priming and polarization of T lymphocytes, the cytokine and chemokine profile in the tumor-derived cell culture supernatants was assessed. In the unstimulated culture supernatants, only three cytokines were detected in significant amounts, IL-6, IL-10 and TNFα (see Fig. 4A). IL-2, IL-4, IL-12, and IL-23 levels were below the limit of detection of the MILLIPLEX^{™} assay in most of the patient samples tested, and IL-17 was only detected in cell supernatants of patients in stage I of disease. Importantly, only minimal concentrations of IL-1β (4.2 ± 2.5 pg/ml), which is crucial for Th17 polarization, were detected in culture supernatants from stage I patients, who had the highest proportions of tumor-infiltrating Th17 cells (see Fig. 4C). Except for IL-17, no other significant correlations with disease progression were observed in the unstimulated cultures. Upon PMA and ionomycin stimulation, most of the tested cytokines were produced, except IL-12 and IL-23 (see Fig. 4A). The production of IL-21 was significantly decreased in culture supernatants from patients in the advanced stages of the disease when compared with culture supernatants from stage I patients (see Fig. 1C). The expression of TGFβ mRNA was observed in all of the patient samples and did not significantly correlate with disease stage (Fig. 4D).

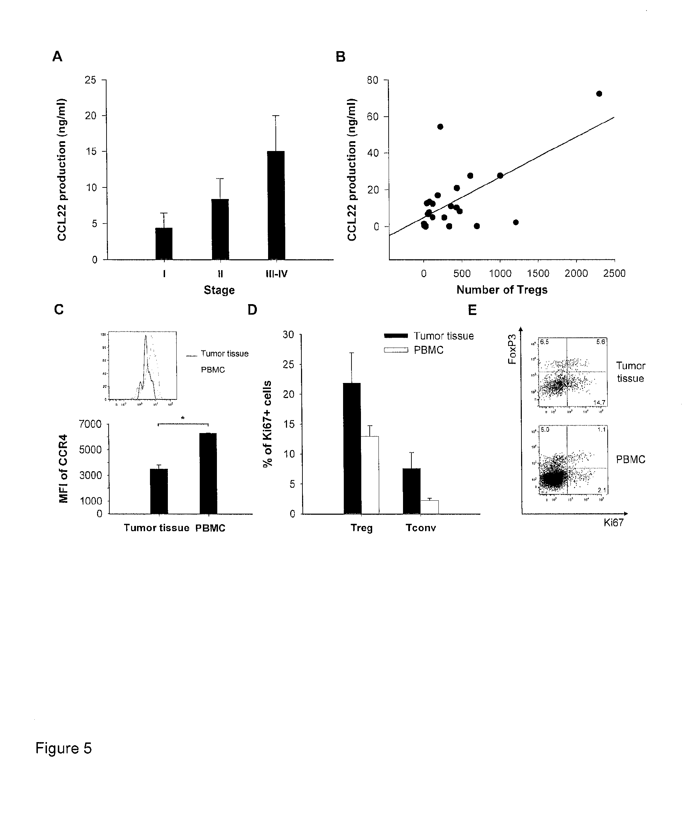

Unstimulated tumor-derived cells produced high amounts of CCL20, CCL22 and inflammatory CXCL9 and CXCL10 (see Fig. 4B) but low amounts of CCL2, CCL5, CCL17, CCL19 and CCL21. The levels of CCL22 (see Fig. 5A), CXCL9 and CXCL10 (not shown) increased during disease progression; however, these data were not statistically significant. The levels of CXCL9 significantly correlated with the numbers of tumor-infiltrating CD4⁺ (r = 0.65, p < 0.01) and CD8⁺ (r = 0.64, p < 0.01) T lymphocytes. There was no correlation with a particular Th lymphocyte subset (not shown). Most important, the levels of CCL22 significantly correlated with the number of Ti-Tregs (r = 0.66, p < 0.01) (Fig. 5B). No other correlations between the chemokines and tumor-infiltrating immune cells were observed.

### E) Tregs are probably recruited to the tumor tissue via a CCL22/CCR4 interaction and proliferate in situ

As described above, the number of Ti-Tregs significantly correlated with the level of CCL22 in the tumor cell culture supernatants. In accord with these observations, Ti-Tregs had significantly lower expression of CCR4 than Tregs from the peripheral blood (mean fluorescence intensity 6,280 ± 35 and 3,487 ± 333, respectively; n = 6) (see Fig. 5C). To evaluate whether Ti- Tregs proliferated *in situ,* Ki67 staining was performed in tumor tissue-derived cell suspensions and PBMCs. A markedly higher percentage of Ti-Tregs expressed Ki67 when compared with Tregs in the peripheral blood (21.8 ± 5.1% and 12.9 ± 1.8%, respectively; n = 6). Ki67 was expressed in a significantly lower proportion of conventional CD4⁺FoxP3⁻ T cells in comparison with Tregs (7.6 ± 2.7% in the tumor tissue and 2.3 ± 0.4% in the peripheral blood) (see Fig. 5D, E).

### F) Ovarian cancer cell lines produce chemokines, including CCL22, upon IFNγ stimulation

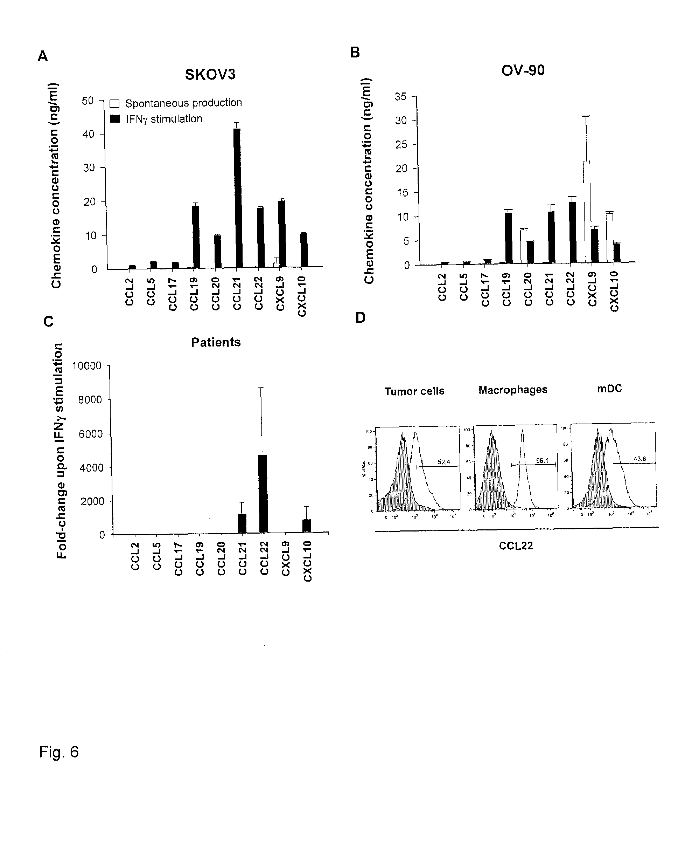

To assess whether ovarian cancer cell lines were able to produce CCL22, OV-90 and SKOV3 cells were cultured either without stimulation or upon stimulation with recombinant human IFNγ, as IFNγ has been shown to induce CCL22 production in breast cancer cell lines. In contrast to primary tumor-derived cell suspensions, spontaneous CCL22 secretion was undetectable in the ovarian cancer cell lines, a result that was similar to most of the other chemokines measured in this study. The SKOV3 cell line spontaneously produced small amounts of CXCL9 (see Fig. 6A), whereas unstimulated OV-90 cells produced high amounts of CXCL9, CXCL10 and CCL20 (see Fig. 6B). The addition of IFNγ induced robust secretion of CCL19, CCL21 and CCL22 in both of the cell lines tested. Surprisingly, production of CXCL9, CXCL10 and CCL20 increased in SKOV3 but decreased in OV-90 cells upon IFNγ stimulation (see Fig. 6A, B).

### G) Primary ovarian tumor-derived cells have markedly increased CCL22 production upon IFNγ stimulation

To evaluate the effect of IFNγ on primary tumor-derived single cell suspensions, cells isolated from patient tumor tissues (n = 6) were cultured in the presence of IFNγ. As shown in Fig. 6C, only CCL21, CCL22 and CXCL10 concentrations increased in the tumor cell culture supernatants upon IFNγ stimulation, with the most significant increase observed for CCL22. As the source of the CCL22 secretion, EpCAM+ tumor cells, macrophages and mDCs were identified in unstimulated as well as stimulated cultures (see Fig. 6D).

The present invention provides an ex vivo method for the diagnosis and/or the monitoring and/or predicting the progress of a cancer disease, whereby in at least one sample the ratio of Tregs-cells to at least one other group of T cells comprising the group of Th17 cells, Th1 cells and/or Th2 cells is determined.

The advantage of the ex vivo method described herein is that the specific type of cancer can be better diagnosed. It is very often decisive which kind of therapeutic agents should be administered to the patient. By better characterizing the type of cancer the therapy can be adapted to the benefit of the patient.

The in vitro method disclosed herein is preferably used for the stratification of patients with ovarian cancer into specific risk groups which can be treated with differentially intensified adjuvant chemotherapy. For the treatment of patients suffering from ovarian cancer it is a substantial advantage to know which type of cancer the patient is suffering from. Whereas in one group of patients it may be advantageous to apply intense chemotherapy, the same type of treatment may have adverse effects in another group of patients. Intravenous carboplatin and paclitaxel is generally considered the standard regime for ovarian cancer. In view of the predominant peritonial surface involvement of metastatic ovarian cancer the active chemotherapeutic agents can also be delivered by an intraperitonial route. Alternatively a platinum-based chemotherapy can be applied. Further chemotherapeutic drugs like liposomal doxorubicin and topotecan or gemcitabine can be used.

An alternative treatment scheme used is iphosphamid, cisplatin and paclitaxel whereby iphosphamid in combination with cisplatin or iphosphamid combined with paclitaxel is preferred. The in vitro method disclosed herein allows a selection of the most effective chemotherapeutic treatment of the specific risk group determined by the ex vivo diagnostic method.

It is also important to monitor the progress of the cancer in the patient depending on the effect of therapies chosen. By the method described herein it is possible to perform the ex vivo diagnostic method and to observe results obtained with a specific treatment. After having received the results of the diagnostic method it can be decided whether the mode of therapy is continued, adapted or changed. Furthermore, it is often desirable to better predict the progress of the cancer disease.

For the present in vitro method a sample is treated in such a manner that the ratio of the Treg cells to Th17 cells or the ratio of Treg cells to TH1 and/or TH2 cells is determined. The determination is performed by methods known to the person skilled in the art. Frequently the cells are identified by antibodies directed against specific surface markers which are characteristic for the cell type population. It is also possible to use several antibodies directed to specific surface markers in combination. Important is, however, that at least one antibody against a surface marker is used which is specific for the particular cell type. For identifying Treg cells it is preferred to use at least one, preferably at least two and more preferred at least three antibodies selected from the group consisting of anti-CD3, anti-CD4, anti-CD8, anti-CD25, anti-CD127 and anti-CR4 and particularly preferred anti-FoxP3 and/or anti-Helios.

The characterization of the cells is usually performed by flow cytometry analysis (Fluorescence Activated Cell Sorting, FACS) which allows a determination of the cell population. In an especially preferred embodiment it is determined whether the cells belong to the Treg cell population or to the Th17 cell population.

The sample which is used in the differentiation method may be derived from a cancer tissue or a metastatic site of cancer tissue. Such samples may for example be removed from the patient after surgery which is common when primary tumors or larger metastatic tumors are removed. Samples may also be obtained by biopsy.

Alternatively the sample may be derived from cell culture which may be derived from tumor tissue. It is possible to mince tumor tissues and separate the tumor cells which are grown under usual conditions. Care has, however, been taken to avoid wrong results caused by the addition of cytokines to the growth medium, which may have an influence on the differentiation of the cells.

For control purposes it may be required to perform the herein described method also on cells obtained from the blood, in particular from the peripheral mononuclear blood cells.

The presence and prognostic value of different types of tumor-infiltrating immune cells within tumor tissues have been extensively studied; however, little is known about the alterations in immune cell patterns within the tumor tissue during disease progression. To investigate the dynamics of immune cell distribution within cancer tissues, adequate selection of patients with both early and advanced disease is essential. However, since only less than 15% of EOC patients are diagnosed at stage I, it is very difficult to study the microenvironment of the early ovarian cancer lesions in prospective studies. Despite the extremely low availability of fresh tumor tissue samples from early disease patients, 6 tumor samples from stage I patients could be analyzed together with 38 tumor samples from patients with more advanced disease (stages II-IV),

The major part of the experiments has been performed with patients suffering from ovarian cancer. It is, however, assumed that the general principle of the change of the ratio of the determined T cells changes analogously with the progress of cancer. Therefore, the method disclosed herein can be applied to all types of cancer, in particular to prostate cancer, breast cancer, colon cancer. Preferred is, however, the use of the in vitro diagnostic method applied to patients suffering from ovarian cancer.

It was found that different subsets of CD4⁺ T cells, but not other immune cell types, were present and exhibited a significantly different pattern during disease progression. Indeed, the early stages of EOC were characterized by a strong Th17 immune response (see Fig. 1A), whereas in advanced stages of disease, a dominant population of regulatory T cells was detected (see Fig. 2A). Surprisingly, CD8⁺ T lymphocytes and pDCs did not show any substantial alterations, whereas the proportions of mDCs and macrophages appeared to increase during disease progression. The proportion of Th1 cells slightly increased in the stage II tumors and then decreased again in the advanced stages of disease. However, neither the alterations in mDCs and macrophages nor those in Th1 cells were statistically significant. A similar kinetics of CD4⁺ effector T cells, i.e., an early burst of Th17 cells that was replaced by high numbers of IFNγ-producing cells, has been described in mouse models of EAE and infection.

Consistent with these observations, Lohr et al. (Microbiol. Infect. 2009, 11, 589-593) transferred antigen-specific T cells into transgenic mice expressing the antigen recognized by the T cells and found the sequential development of Th17 and then Th1 effector cells followed by Treg development in the late phase of disease. This dynamic has not yet been described in the microenvironment of human tumors and it was uncertain whether test results obtained in the mouse model can be transferred to humans.

Notably, the origin of the tumor-infiltrating Th17 cells and their exact role in tumor immunity remain controversial. Kryczek et al. [Blood (2009), 114, 1141-1149] has reported that macrophages isolated from EOC tissues are capable of inducing Th17 cells *in vitro.* However, because IL-1β, IL-6 and IL-23 are supposed to be essential for the differentiation of human Th17 cells, the cytokine profile of the tumor microenvironment that was observed in the present invention, especially the very low levels of IL-1β in stage I patients together with the complete lack of IL-23 (see Fig. 4C), does not support the hypothesis of Th17 polarization *in situ.* It has been shown that Th17 trafficking could be mediated via the CCR6/CCL20 axis. Surprisingly, in the present study no correlation between the numbers of Th17 cells and CCL20 levels in the tumor tissue has been observed.

In comparison with Th17 cells, many more studies have focused on the trafficking properties and prognostic significance of Tregs in tumors. Curiel et al. [Nat. Med. (2004) 10, 942-949] have shown that Tregs infiltrating ovarian cancer tissues effectively inhibit TAA-specific immunity *in vitro* and *in vivo* and contribute to tumor growth. These authors also suggested that Treg tumor trafficking could be mediated by CCL22. Treg recruitment into the tumor tissue via CCL22/CCR4 has recently been confirmed in breast cancer patients.

In the present study it has been shown that Ti-Tregs were mainly Helios⁺ activated Tregs. A significant number of these Ti-Tregs was demethylated in the *FoxP3* TSDR. Naturally occurring, but not *in vitro* TGFβ-induced, Foxp3⁺ Tregs have been shown to display stable Foxp3 expression that is associated with selective demethylation of the TSDR. Similarly, the Ikaros family member, Helios, has been reported to be a marker useful for discriminating between naturally occurring thymic-derived Tregs (nTregs) and those peripherally induced from naïve CD4⁺ T cells (iTregs), but these findings have very recently been questioned. Thus, rather than discriminating between nTregs and iTregs, Helios appears to be useful for indicating activated Tregs regardless of their origin. Taken together, the high expression of Helios together with the demethylation of the TSDR found in the Ti-Tregs in the EOC tissues as disclosed herein indicate the stability and highly activated status of these cells.

In accord with the studies concerning Treg tumor trafficking, it has been demonstrated that the number of Ti-Tregs significantly correlated with the concentration of CCL22 in the ovarian tumor cell culture supernatants. Consistently, Ti-Tregs had significantly lower expression of CCR4 than circulating Tregs. Low levels of CCR4 reflect its internalization due to an active engagement with CCL22. It was also observed that a large proportion of Ti-Tregs (21.8 ± 5.1 %) expressed the proliferation marker, Ki67, indicating their extensive local expansion.

Faget et al. [Cancer Research (2011), 71, 6143-6152] have demonstrated that IFNγ triggers production of CCL22 by breast tumor cells. Indeed, the cooperation between tumor cells and the immune cell infiltrate (especially macrophages and NK cells) has been shown to be essential for inducing high quantities of CCL22. Similarly, it was observed that ovarian cancer cell lines produced chemokines, including significant levels of CCL22, upon IFNγ stimulation. Most important, in primary ovarian cancer tissue-derived cells, recombinant IFNγ selectively augmented CCL22 production with only a small effect on the other chemokines tested (see Fig. 6C). Tumor cells, macrophages and mDCs were identified as the key source of CCL22 in the cancer tissue samples in our study.

In summary, the experimental data suggest that the development of the anti-tumor immune response in ovarian cancer patients is highly dynamic. In the early stages of the disease, a strong recruitment of Th17 cells is observed, which is followed by a recruitment of Th1 cells, macrophages and mDCs. In the later stages of EOC, enhanced levels of IFNγ, probably produced by the immigrating cells, together with increased proportions of macrophages and mDCs may favor the production of CCL22 and the recruitment of Tregs via CCL22/CCR4, thus promoting the development of a suppressive microenvironment. Thus, as the disclosed findings clearly show a dynamic shift from an active anti-tumor immune response to a significant immune suppression in the advanced EOC, the development of novel cancer immunotherapy protocols should not only involve immune-boosting strategies but should also target the immunosuppressive tumor microenvironment in advanced disease.

The results of the experiments are summarized in the Figures:

### Figure 1

Proportions of Th17 cells within the primary ovarian tumor tissue and peripheral blood according to the stage of the disease are shown as % Th17 lymphocytes related to all CD4⁺ T cells.
(A) Data are expressed as the proportion of Th17 lymphocytes among CD4⁺ T cells, and represent the mean value in each stage.
(B) Dot plots are gated on CD3⁺CD4⁺ cells and show IL-17 and IFNγ in two representative patients.
(C) Columns represent mean levels of IL-17 and IL-21 produced by PMA + ionomycin stimulated tumor tissue-derived cells + S.E.M.
(D) Columns represent mean proportions of Th17 cells in the tumor tissue and peripheral blood mononuclear cells (PBMC) + S.E.M. * p < 0.05, ** p < 0.01 (A, ANOVA followed by Scheffé test; D, paired t-test).

### Figure 2

Proportions of Tregs within the primary ovarian tumor tissue and peripheral blood according to the stage of the disease.
(A) Data are expressed as the proportion of CD4⁺CD25⁺CD127⁻FoxP3⁺ Tregs among CD4⁺ T cells, and represent the mean value in each stage.
(B) Dot plots are gated on CD3⁺CD4⁺ cells and show CD25⁺FoxP3⁺ Tregs in 2 representative patients.
(C) Columns represent mean proportions of Tregs in the tumor tissue and PBMC + S.E.M.
(D) Dot plots are gated on CD3⁺CD4⁺ CD25⁺CD127⁻ cells and show FoxP3⁺Helios⁺ Tregs in the tumor tissue of a representative patient. Box plots represent the proportion of Helios⁺ Tregs among CD4⁺CD25⁺CD127⁻FoxP3⁺ Tregs. The boundaries of the box indicate the 25th and the 75th percentiles, the line within the box represents the median. Whiskers indicate the 90th and 10th percentiles.
(E) Columns represent proportions of Tregs demethylated in *FoxP3* TSDR among cells isolated from the primary ovarian tumor tissue in different stages of the disease, error bars indicate S.E.M.
(F) The line shows negative correlation between % of tumor-infiltrating Tregs and Th17 cells.
(G) The line shows negative correlation between % of tumor-infiltrating Tregs and the number of tumor-infiltrating macrophages. * p < 0.05, ** p < 0.01 (A, ANOVA followed by Scheffé test; C, D, paired t-test).

### Figure 3

Proportions of Th1 lymphocytes, myeloid Dendritic Cells (mDCs) and macrophages within the primary ovarian tumor tissue according to the stage of the disease. (A) Data are expressed as the mean proportion of CD3+CD4⁺IFNγ+ Th1 cells among CD4⁺ T cells + S.E.M. (B) Columns represent the mean number of CD45⁺Lineage⁻HLA-DR⁺CD14-CD11c⁺ mDCs in 1 M of isolated tumor-derived cells + S.E.M. (C) Columns represent the mean number of CD45⁺Lineage-HLA-DR⁺CD14⁺ macrophages in 1 M of isolated tumor-derived cells + S.E.M.

### Figure 4

Cytokine and chemokine profiles of tumor-derived single cell suspensions. (A) White columns represent the mean spontaneous cytokine production; black columns represent cytokine production upon PMA and ionomycin stimulation. (B) Columns represent mean spontaneous chemokine production. All error bars indicate S.E.M. (C) The plot expresses the dynamics of the mean spontaneous production of Th17 polarizing cytokines and IL-17 during the disease progression. (D) The plot indicates the dynamics of TGFβ mRNA expression during the disease progression presented as the mean proportion of β-actin mRNA expression.

### Figure 5

Recruitment of Tregs into the ovarian tumor tissue is probably CCL22-mediated. (A) Columns represent CCL22 (macrophage-derived chemokine) production in tumor tissue derived cell culture supernatants + S.E.M. (B) The scatter plot expresses the positive correlation between CCL22 production and number of Ti-Tregs. (C) Histograms show the CCR4 expression in CD4⁺CD25⁺CD127⁻FoxP3⁺ Tregs in the tumor tissue (black line) and peripheral blood (gray line, tinted) in a representative patient. Columns represent the mean fluorescence intensity of CCR4 on CD4⁺CD25⁺CD127⁻FoxP3⁺ Tregs in the tumor tissue and peripheral blood + S.E.M. (n = 6). (D) Columns represent the mean proportion of proliferating Ki67⁺CD4⁺CD25⁺CD127⁻FoxP3⁺ Tregs and CD4⁺FoxP3⁻ conventional T cells (Tconv) in the tumor tissue and peripheral blood + S.E.M. (n = 6). (E) Dot plots are gated on CD3⁺CD4⁺ cells and show the proportion of Ki67⁺ FoxP3⁺ Tregs and FoxP3⁻ Tconv in a representative stage III patient. * p < 0.05 (paired t-test).

### Figure 6

IFNγ induces chemokine production in both ovarian cancer cell lines and patients. (A, B) Columns represent the mean chemokine production in ovarian cancer cell lines with (black columns) or without IFNγ (white columns) stimulation + S.E.M. (C) Data are expressed as the mean fold-changes in chemokine levels in IFNγ-stimulated primary tumor-derived cell cultures in comparison to unstimulated cultures + S.E.M. (D) Histograms show the spontaneous expression of CCL22 in primary tumor-derived cells. Cells are gated on CD45⁻EpCAM⁺ tumor cells; CD45⁺Lineage⁻HLA⁻DR⁺CD14⁺ macrophages and CD45⁺Lineage⁻HLA⁻DR⁺CD14⁻CD11c⁺ mDCs.

### Example 1

### a) Patients and tissue samples

Peripheral blood and primary epithelial ovarian cancer specimens were obtained from 44 patients undergoing initial cytoreductive surgery at the University Hospital Motol in Prague between March 2009 and October 2011. None of the patients enrolled in the study had received neoadjuvant chemotherapy prior to the surgery. All tissue specimens were collected with patient consent, and the study was approved by the Institutional Review Board of the University Hospital Motol.

**Table 1**

| **Variable** | | **No.** | **%** |
|---|---|---|---|
| Total no. of patients | | 44 | |
| Age | | | |
| | Mean | 56 | |
| | Range | 33-83 | |
| FIGO stage | | | |
| | I | 6 | 13.6 |
| | II | 5 | 11.4 |
| | III | 32 | 72.7 |
| | IV | 1 | 2.3 |
| Histological subtype | | | |
| | Serous | 27 | 61.4 |
| | Mucinous | 9 | 20.4 |
| | Clear cell | 3 | 6.8 |
| | Other | 5 | 11.4 |
| Differentiation | | | |
| | Well | 6 | 13.6 |
| | Moderate | 9 | 20.4 |
| | Poor | 21 | 47.7 |
| | ND | 8 | 18.3 |

Table 1 shows the clinicopathological characteristics of the EOC patients.

Tumor tissue was minced with scissors, digested in PBS containing 1 mg/ml of Collagenase D (Roche, Basel, Switzerland) at 37 °C for 30 min, mechanically dissociated using the gentleMACS^{™} Dissociator (Miltenyi Biotec, Auburn, CA) and passed through a 100 µm nylon cell strainer (BD Biosciences, Franklin Lakes, NJ). Peripheral blood mononuclear cells (PBMCs) from blood samples were isolated using Ficoll-Paque density gradient solution (GE Healthcare, Waukesha, WI).

### b) Cell lines

The ovarian cancer cell lines, OV-90 and SKOV3, were cultured in RPMI 1640 supplemented with 10% heat-inactivated FCS, L-glutamine and penicillin-streptomycin (all from Invitrogen, Carlsbad, CA) at 37 °C and 5% CO₂.

### Example 2 - Analytical methods used

### 2.1 Flow cytometry analysis

To detect tumor infiltrating myeloid DCs (mDCs), plasmacytoid DCs (pDCs) and macrophages, single cell suspensions were stained with specific antibodies against CD3, CD11c, CD14, CD16, CD19, CD20, CD45, CD56 (Exbio, Vestec, Czech Republic), CD123 (eBioscience, San Diego, CA) and HLA-DR (BD Biosciences). Regulatory T cells (Tregs) were identified by surface staining with anti-CD3 (Exbio), anti-CD4 (eBioscience), anti-CD8 (Exbio), anti-CD25, anti-CD127 and anti-CCR4 (BioLegend) antibodies followed by fixation and permeabilization with a Foxp3 Staining Buffer Set (eBioscience) and intracellular staining with anti-FoxP3 (eBioscience) and anti-Helios (BioLegend) antibodies. For detection of intracellular cytokines, cell suspensions were stimulated with 50 ng/ml of PMA and 1 µg/ml of ionomycin (Sigma-Aldrich, St. Louis, MO) for 4 h in the presence of brefeldin A (BioLegend). After the 4 h incubation, cells were stained with antibodies against CD3 (Exbio), CD4 (eBioscience), and CD8 (Exbio), fixed and permeabilized with the Foxp3 Staining Buffer Set (eBioscience) and stained with anti-IL-17 (BioLegend) and anti-IFNγ (BD Biosciences) antibodies. For CCL22 detection, cells were cultured in the presence of brefeldin for 4 h and labeled with the antibodies used for DC/macrophage identification, EpCAM (BioLegend) and CCL22 (R&D, Minneapolis, MN) as described above. Samples were analyzed on a BD FACSAria (BD Biosciences) using FlowJo software (TreeStar, Ashland, OR).

### 2.2 ELISA analysis

In a similar manner as described above the cells have been identified and differentiated by using suitable antibodies in an ELISA format. By using antibodies directed against surface markers of the T cells it is possible to easily differentiate the cells.

### 2.3 Real time PCR

In another alternative embodiment the differentiation of the cells can be performed by real time PCR. In this method primers specific for characteristic genes were used which show the expression of the relevant surface markers. By using suitable primer combinations it can be determined in a simple PCR test which proportion of Treg cells compared to Th17 cells is in the sample.

### 2.4 Cytokine and chemokine detection

Tumor tissue-derived cell suspensions (1 x 10⁶ cells/ml) were cultured in RPMI 1640 supplemented with 10% heat-inactivated FCS, L-glutamine and penicillin-streptomycin (Invitrogen) in the presence or absence of PMA + ionomycin. For IFNγ-mediated induction of chemokines, ovarian cancer cell lines and primary tumor tissue-derived cell suspensions (1 x 10⁶ cells/ml) were cultured in the presence of 10, 50 and 100 ng/ml of recombinant human IFNγ (Invitrogen). After 24 h of incubation, culture supernatants were harvested and stored at -80 °C until use. Concentrations of IL-1β, IL-2, IL-4, IL-6, IL-7, IL-10, IL-12(p70), IL-13, IL-17a, IL-21, IFNγ, and TNFα released into the culture supernatants were determined using a MILLIPLEX^{™} Human Cytokine/Chemokine Kit (Millipore, Billerica, MA). Concentrations of chemokines (CCL2, CCL5, CCL17, CCL19, CCL20, CCL21, CCL22, CXCL9, and CXCL10) were analyzed using a Quantibody^{®} Array Kit (Raybiotech, Norcross, GA).

### 2.5 Extraction of genomic DNA and quantitative real time PCR-based methylation assay

Genomic DNA (gDNA) was isolated from a lysate containing 2 x 10⁶ tumor tissue-derived cells using the PureLink Genomic DNA Mini kit (Invitrogen, Carlsbad, USA). Concentrations of extracted gDNA were measured with a Nanodrop^{©} 2000c UV-Vis spectrophotometer (Thermo Scientific, Waltham, USA). Four to five micrograms of gDNA was treated with sodium bisulfite using the MethylCode Bisulfite Conversion kit (Invitrogen). Quantitative real time PCR amplification of the methylated and demethylated *FoxP3* Treg-specific demethylated region (TSDR) was performed using bisulfite-treated gDNA, 0.5 U Platinum Taq DNA polymerase (Invitrogen), PCR buffer without MgCl₂, 3 mM MgCl₂, 0.2 mM dNTPs (each), 1 µM primers and 0,2 µM TaqMan^{©} probe. PCR reactions were performed using a CFX 96 cycler (BioRad, Hercules, USA). Methylation-specific and demethylation-specific primers and TaqMan© probes were commercially synthesized (TIB MOLBIOL, Berlin, Germany). Amounts of methylated and demethylated *FoxP3 TSDR* DNA were estimated from calibration curves by crossing points linear regression using the second derivative maximum method as described by Rasmussen et al. [Meuer, Wittwer, Nagawara (2001), Rapid Cycle Real-time PCR, Springer, pp. 21-34]. The proportion of cells with a demethylated *FoxP3* TSDR was calculated as the ratio of demethylated *FoxP3* TSDR DNA to the sum of methylated and demethylated *FoxP3* TSDR DNA [Wiezorek et al., Cancer Res. (2009), 69, 599-608 and de Vries et al., Clin. Cancer Res. (2011), 17, 841-848].

### 2.6 Plasmid constructs

DNA fragments of the methylated and demethylated *FoxP3* TSDR (86 bp) were cloned into the pUC18 plasmid (Amersham Biosciences, Amersham, UK) using *Hind*III and EcoRI restriction sites. The identity of the plasmid constructs was verified by sequencing followed by plasmid DNA purification using a Wizard Plus Midipreps kit (Promega, Madison, USA) and dilution to seven final concentrations of 100, 10, and 1 pg/µl and 100, 10, 1 and 0,1 fg/µl, representing a range of 3,34 x 10⁻⁷ to 3,34 x 10⁻¹ plasmid copies. Serial dilutions demonstrated the specificity of the assay and were used as standards for quantification of the methylated and demethylated *FoxP3* TSDR.

### 2.7 RNA extraction and quantitative real time PCR

Total RNA was extracted from 2 x 10⁶ tumor-tissue derived cells using an RNA Easy Mini Kit (Qiagen, Hilden, Germany). RNA concentrations were determined with a Nanodrop^{©} 2000c UV-Vis spectrophotometer (Thermo Scientific), and RNA integrity was assessed using an Experion automated system (BioRad). Complementary DNA was synthesized from total RNA using the M-MLV reverse transcriptase (Invitrogen) and amplified by quantitative real time PCR in the presence of primers and TaqMan^{©} probes specific for TGFβ and the β-actin housekeeping gene, which was used as an internal reference. All primers and probes were commercially synthesized (TIB MOLBIOL). The identity of qPCR products in each assay was verified by sequencing. The relative expression of the target genes was normalized to the expression of β-actin.

### 2.8 Statistical analysis

Statistical analyses were performed using Statistica® 10.0 software (StatSoft, Tulsa, OK). The parametric assumptions of the data were verified using the Kolmogorov-Smirnov test for normality. The homogeneity of variances was tested by the Levene test. Correlations between tumor-infiltrating immune cells were evaluated using the Pearson r coefficient. The differences between tumor tissue and peripheral blood samples were analyzed using a paired t-test. The remaining data were analyzed using an ANOVA followed by a Scheffé test. The results were considered statistically significant when p < 0.05.

## Claims

1. In vitro method for the diagnosis and/or the monitoring and/or predicting the progress of a cancer disease, **characterized in that** in at least one sample the ratio of Tregs-cells to at least one other group of T cells comprising the group of Th17 cells, Th1 cells and/or Th2 cells is determined.

2. In vitro method according to claim 1, wherein the diagnosis is used for the stratification of patients into specific risk groups.

3. In vitro method according to claim 1, **characterized in that** the ratio of Tregs cells to Th17 cells is determined.

4. In vitro method according to claim 1 or 2, **characterized in that** the sample is derived from a tumor or a cell culture derived from a tumor.

5. In vitro method according to any of claims 1-3, **characterized in that** the ratio of the Treg cells is determined by flow cytometry analysis.

6. In vitro method according to claim 4, **characterized in that** the flow cytometry analysis of the Tregs cells is performed by surface staining with at least one of the antibodies selected from the group consisting of anti-CD3, anti-CD4, anti-CD8, anti-CD25, anti-CD127, anti-CR4, anti-FoxP3 and anti-Helios.

7. In vitro method according to any of claims 1-3, **characterized in that** the ratio of the T reg cells compared with other T cells is determined by ELISA.

8. In vitro method according to any of claims 1-3, **characterized in that** the percentage of Treg cells is determined by real time PCR.
